# EUROPEAN PATENT SPECIFICATION

(11) **EP 2 943 179 B1**
(45) Date of publication and mention of the grant of the patent: **06.03.2019**
(21) Application number: 14700159.8
(22) Date of filing: 08.01.2014
(51) Int. Cl.: A61K 8/31, A61K 8/46, A61K 8/60, A61Q 5/10, A61K 8/73

(54) **DYEING COMPOSITION FREE OF CHEMICAL OXIDIZING AGENT, COMPRISING AN OXIDATION DYE, AN ALKYL SULFATE, AN ALKYLPOLYGLYCOSIDE, A FATTY SUBSTANCE AND NON-IONIC GUAR GUM**
VON CHEMISCHEN OXIDATIONSMITTELN FREIES FÄRBEMITTEL MIT EINEM OXIDATIONSFARBSTOFF, EINEM ALKYLSULFAT, EINEM ALKYLPOLYGLYCOSID, EINER FETTSUBSTANZ UND EINEM NICHTIONISCHEN GUARKAUTSCHUK
COMPOSITION DE COLORATION EXEMPTE D'AGENT D'OXYDATION CHIMIQUE, COMPRENANT UN COLORANT D'OXYDATION, UN ALKYLSULFATE, UN ALKYLPOLYGLYCOSIDE, UNE SUBSTANCE GRASSE ET UNE GOMME DE GUAR NON IONIQUE

(30) Priority: 09.01.2013 FR 1350184; 25.02.2013 US 201361768583 P
(43) Date of publication of application: 18.11.2015
(73) Proprietor: L'Oréal, 75008 Paris (FR)
(72) Inventor: CHARRIER, Delphine, F-92100 Boulogne Billancourt (FR)
(74) Representative: Kuhlmann, Sonia
(86) International application number: PCT/EP2014/050210
(87) International publication number: WO 2014/108433

(56) References cited:
- EP-A2- 1 123 693
- DE-A1-102011 017 519
- US-A- 4 804 385

## Description

The present invention relates to a dyeing composition which is free of chemical oxidizing agent other than atmospheric oxygen and which comprises oxidation dye precursors and a non-ionic guar gum.

Among the methods for dyeing human keratin fibres, such as the hair, mention may be made of oxidation dyeing or permanent dyeing. More particularly, this form of dyeing uses one or more oxidation dyes, usually one or more oxidation bases optionally combined with one or more couplers.

In general, the oxidation bases are chosen from ortho- or para-phenylenediamines, ortho- or para-aminophenols and heterocyclic compounds. These oxidation bases are colourless or weakly coloured compounds which, when combined with oxidizing products, can give access to coloured entities.

The shades obtained with these oxidation bases are often varied by combining them with one or more couplers, these couplers being chosen in particular from aromatic meta-diamines, meta-aminophenols, meta-diphenols and certain heterocyclic compounds, such as indole compounds.

The variety of molecules used as oxidation bases and couplers allows a wide range of colours to be obtained.

Permanent dyeing processes consist in using, with the dye composition, an aqueous composition comprising at least one oxidizing agent, such as hydrogen peroxide, under alkaline pH conditions in the vast majority of cases. The alkaline agent conventionally used is aqueous ammonia or other alkaline agents, such as alkanolamines.

The dyeing compositions applied to the fibres, which contain the oxidizing agent, may be in various forms such as lotions, gels, emulsions, creams or foams.

Document US4804385A discloses in example 8 a composition for dyeing human keratin fibres which is free of chemical oxidizing agent other than atmospheric oxygen and which comprises dihydroxyindole, non ionic guar gum and a glycoside alkyl ether.

There is a real need to develop oxidation dyeing compositions which, firstly, before being mixed with the oxidizing composition, have suitable rheology, and which, after mixing with the oxidizing agent, produce a good texture, which remains sufficiently stable over time, with good working qualities once applied, while at the same time retaining efficient dyeing properties, in particular in terms of strength and homogeneity of the colouration obtained.

These objectives are achieved by the present invention, a subject of which is a composition for dyeing human keratin fibres which is free of chemical oxidizing agent other than atmospheric oxygen and which comprises:
(a) at least one oxidation dye precursor;
(b) at least one liquid fatty substance;
(c) at least one non-ionic guar gum;
(d) at least one anionic surfactant chosen from C₆-C₄₀ alkyl sulfates and non-salified forms thereof;
(e) at least one non-ionic alkylpolyglycoside surfactant.

It also relates to a process for dyeing human keratin fibres, in which a composition according to the invention is applied to the fibres in the presence of at least one chemical oxidizing agent other than atmospheric oxygen.

The composition of the invention exhibits satisfactory rheology in terms of viscosity and stability.

The composition of the invention makes it possible to obtain, by extemporaneous mixing with a composition comprising at least one chemical oxidizing agent other than atmospheric oxygen, a composition which is particularly pleasant to apply. This mixture composition in fact has an aerated, foaming texture which makes it particularly easy to distribute over the hair. The qualities of this mixture are, moreover, sufficiently persistent over time to allow homogeneous application of the dyeing product without running and to keep it in place during the leave-on time.

The composition of the invention also makes it possible to obtain good, particularly effective dyeing properties, such as strength and homogeneity of the colour, and resistance to external agents (shampoo, perspiration, light).

Other characteristics and advantages of the invention will emerge more clearly on reading the description and the examples that follow.

In the text hereinbelow, and unless otherwise indicated, the limits of a range of values are included in that range.

The term "at least one" associated with an ingredient of the composition signifies "one or more".

The human keratin fibres treated by means of the process according to the invention are preferably the hair.

### Dyes

As indicated previously, the dye composition according to the invention comprises at least one oxidation dye precursor.

As oxidation dye precursors, use may be made of oxidation bases and couplers. Preferably, the composition contains at least one oxidation base. More preferably, the composition contains at least one oxidation base and at least one coupler.

By way of example, the oxidation bases are chosen from para-phenylenediamines, bis(phenyl)alkylenediamines, para-aminophenols, ortho-aminophenols and heterocyclic bases, and the addition salts thereof.

Among the para-phenylenediamines that may be mentioned, for example, are para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine, N-(β-methoxyethyl)-para-phenylenediamine, 4-aminophenylpyrrolidine, 2-thienyl-para-phenylenediamine, 2-β-hydroxyethylamino-5-aminotoluene and 3-hydroxy-1-(4'-aminophenyl)pyrrolidine, and the addition salts thereof with an acid.

Among the para-phenylenediamines mentioned above, para-phenylenediamine, para-tolylenediamine, 2-isopropyl-para-phenylenediamine, 2-β-hydroxyethyl-para-phenylenediamine, 2-β-hydroxyethyloxy-para-phenylenediamine, 2,6-dimethyl-paraphenylenediamine, 2,6-diethyl-para-phenylenediamine, 2,3-dimethyl-para-phenylenediamine, N,N-bis(β-hydroxyethyl)-para-phenylenediamine, 2-chloro-para-phenylenediamine and 2-β-acetylaminoethyloxy-para-phenylenediamine, and the addition salts thereof with an acid, are particularly preferred.

Among the bis(phenyl)alkylenediamines that may be mentioned, for example, are N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(P-hydroxyethyl)-N,N'-bis(4-aminophenyl)tetramethylenediamine, N,N'-bis(4-methylaminophenyl)-tetramethylenediamine, N,N'-bis(ethyl)-N,N'-bis(4'-amino-3'-methylphenyl)ethylenediamine, 1,8-bis(2,5-diaminophenoxy)-3,6-dioxaoctane, and the addition salts thereof.

Among the para-aminophenols that may be mentioned, for example, are para-aminophenol, 4-amino-3-methylphenol, 4-amino-3-fluorophenol, 4-amino-3-chlorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

Among the ortho-aminophenols that may be mentioned, for example, are 2-aminophenol, 2-amino-5-methylphenol, 2-amino-6-methylphenol and 5-acetamido-2-aminophenol, and the addition salts thereof.

Among the heterocyclic bases, mention may be made, by way of example, of pyridine derivatives, pyrimidine derivatives and pyrazole derivatives.

Among the pyridine derivatives that may be mentioned are the compounds described, for example, in patents GB 1 026 978 and GB 1 153 196, for instance 2,5-diaminopyridine, 2-(4-methoxyphenyl)amino-3-aminopyridine and 3,4-diaminopyridine, and the addition salts thereof.

Other pyridine oxidation bases that are useful in the present invention are the 3-aminopyrazolo[1,5-a]pyridine oxidation bases or the addition salts thereof described, for example, in patent application FR 2 801 308. Mention may be made, by way of example, of pyrazolo[1,5-a]pyrid-3-ylamine, 2-(acetylamino)pyrazolo[1,5-a]pyrid-3-ylamine, 2-(morpholin-4-yl)pyrazolo[1,5-a]pyrid-3-ylamine, 3-aminopyrazolo[1,5-a]pyridine-2-carboxylic acid, 2-methoxypyrazolo[1,5-a]pyrid-3-ylamine, (3-aminopyrazolo[1,5-a]pyrid-7-yl)methanol, 2-(3-aminopyrazolo[1,5-a]pyrid-5-yl)ethanol, 2-(3-aminopyrazolo[1,5-a]pyrid-7-yl)ethanol, (3-aminopyrazolo[1,5-a]pyrid-2-yl)methanol, 3,6-diaminopyrazolo[1,5-a]pyridine, 3,4-diaminopyrazolo[1,5-a]pyridine, pyrazolo[1,5-a]pyridine-3,7-diamine, 7-(morpholin-4-yl)pyrazolo[1,5-a]pyrid-3-ylamine, pyrazolo[1,5-a]pyridine-3,5-diamine, 5-(morpholin-4-yl)pyrazolo[1,5-a]pyrid-3-ylamine, 2-[(3-aminopyrazolo[1,5-a]pyrid-5-yl)(2-hydroxyethyl)amino]ethanol, 2-[(3-aminopyrazolo[1,5-a]pyrid-7-yl)(2-hydroxyethyl)amino]ethanol, 3-aminopyrazolo[1,5-a]pyridin-5-ol, 3-aminopyrazolo[1,5-a]pyridin-4-ol, 3-aminopyrazolo[1,5-a]pyridin-6-ol, 3-aminopyrazolo[1,5-a]pyridin-7-ol and the addition salts thereof.

Among the pyrimidine derivatives that may be mentioned are the compounds described, for example, in patents DE 2359399, JP 88-169571, JP 05-63124 and EP 0 770 375 or patent application WO 96/15765, such as 2,4,5,6-tetraaminopyrimidine, 4-hydroxy-2,5,6-triaminopyrimidine, 2-hydroxy-4,5,6-triaminopyrimidine, 2,4-dihydroxy-5,6-diaminopyrimidine, 2,5,6-triaminopyrimidine and the addition salts thereof, and the tautomeric forms thereof, when a tautomeric equilibrium exists.

Among the pyrazole derivatives that may be mentioned, for example, are the compounds described in the patents DE 3843892, DE 4133957 and patent applications WO 94/08969, WO 94/08970, FR-A-2 733 749 and DE 195 43 988, such as 4,5-diamino-1-methylpyrazole, 4,5-diamino-1-(P-hydroxyethyl)pyrazole, 3,4-diaminopyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-*tert*-butyl-1-methylpyrazole, 4,5-diamino-1-*tert*-butyl-3-methylpyrazole, 4,5-diamino-1-(β-hydroxyethyl)-3-methylpyrazole, 4,5-diamino-1-ethyl-3-methylpyrazole, 4,5-diamino-1-ethyl-3-(4'-methoxyphenyl)pyrazole, 4,5-diamino-1-ethyl-3-hydroxymethylpyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2'-aminoethyl)amino-1,3-dimethylpyrazole, 3,4,5-triaminopyrazole, 1-methyl-3,4,5-triaminopyrazole, 3,5-diamino-1-methyl-4-methylaminopyrazole, 3,5-diamino-4-(β-hydroxyethyl)amino-1-methylpyrazole, and the addition salts thereof. Use may also be made of 4,5-diamino-1-(β-methoxyethyl)pyrazole.

Use will preferably be made of a 4,5-diaminopyrazole and even more preferentially of 4,5-diamino-1-(β-hydroxyethyl)pyrazole and/or one of the addition salts thereof.

Mention may also be made, as pyrazole derivatives, of diamino-N,N-dihydropyrazolopyrazolones and in particular those described in application FR-A-2 886 136, such as the following compounds and the addition salts thereof: 2,3-diamino-6,7-dihydro-1H,5H-pyrazolo[1,2-a]pyrazol-1-one, 2-amino-3-ethylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-dimethyl-1,2-dihydropyrazol-3-one, 4,5-diamino-1,2-diethyl-1,2-dihydropyrazol-3-one, 4,5-diamino-1,2-di(2-hydroxyethyl)-1,2-dihydropyrazol-3-one, 2-amino-3-(2-hydroxyethyl)amino-6,7-dihydro-1H,5H-pyrazolo[1,2-a]pyrazol-1-one, 2-amino-3-dimethylamino-6,7-dihydro-1H,5H-pyrazolo[1,2-a]pyrazol-1-one, 2,3-diamino-5,6,7,8-tetrahydro-1H,6H-pyridazino[1,2-a]pyrazol-1-one, 4-amino-1,2-diethyl-5-(pyrrolidin-1-yl)-1,2-dihydropyrazol-3-one, 4-amino-5-(3-dimethylaminopyrrolidin-1-yl)-1,2-diethyl-1,2-dihydropyrazol-3-one or 2,3-diamino-6-hydroxy-6,7-dihydro-1H,5H-pyrazolo[1,2-a]pyrazol-1-one. Use is preferably made of 2,3-diamino-6,7-dihydro-1H,5H-pyrazolo[1,2-a]pyrazol-1-one and/or an addition salt thereof.

Heterocyclic bases that will preferentially be used include 4,5-diamino-1-(β-hydroxyethyl)pyrazole and/or 2,3-diamino-6,7-dihydro-1H,5H-pyrazolo[1,2-a]pyrazol-1-one and/or an addition salt thereof.

Among the couplers that may be used in the composition according to the invention, mention may be made in particular of meta-phenylenediamines, meta-aminophenols, meta-diphenols, naphthalene-based couplers and heterocyclic couplers, for instance indole derivatives, indoline derivatives, sesamol and derivatives thereof, pyridine derivatives, pyrazolotriazole derivatives, pyrazolones, indazoles, benzimidazoles, benzothiazoles, benzoxazoles, 1,3-benzodioxoles, quinolines, and the addition salts.

These couplers are more particularly chosen from 2,4-diamino-1-(β-hydroxyethyloxy)benzene, 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2-amino-4-(β-hydroxyethylamino)-1-methoxybenzene, 1,3-diaminobenzene, 1,3-bis(2,4-diaminophenoxy)propane, sesamol, 1-amino-2-methoxy-4,5-methylenedioxybenzene, α-naphthol, 6-hydroxyindole, 4-hydroxyindole, 4-hydroxy-N-methylindole, 6-hydroxyindoline, 2,6-dihydroxy-4-methylpyridine, 1H-3-methylpyrazol-5-one, 1-phenyl-3-methylpyrazol-5-one, 2-amino-3-hydroxypyridine, 3,6-dimethylpyrazolo[3,2-c]-1,2,4-triazole and 2,6-dimethylpyrazolo[1,5-b]-1,2,4-triazole, the addition salts thereof, and mixtures thereof.

The addition salts of the oxidation bases and couplers are in particular chosen from the addition salts with an acid, such as the hydrochlorides, hydrobromides, sulfates, citrates, succinates, tartrates, lactates, tosylates, benzenesulfonates, phosphates and acetates.

The oxidation base(s) are generally each present in an amount from 0.0001% to 10% by weight relative to the total weight of the dye composition and preferably from 0.005% to 5% by weight relative to the total weight of the composition.

The coupler(s) each generally represent from 0.0001% to 10% by weight relative to the total weight of the composition and preferably from 0.005% to 5% by weight relative to the total weight of the dye composition.

The dye composition used according to the invention may optionally comprise synthetic or natural, cationic or non-ionic, direct dyes.

Examples of particularly suitable direct dyes that may be mentioned include nitrobenzene dyes; azo direct dyes; azomethine direct dyes; methine direct dyes; azacarbocyanin direct dyes, for instance tetraazacarbocyanins (tetraazapentamethines); quinone and in particular anthraquinone, naphthoquinone or benzoquinone direct dyes; azine direct dyes; xanthene direct dyes; triarylmethane direct dyes; indoamine direct dyes; indigoid direct dyes; phthalocyanine direct dyes, porphyrin direct dyes and natural direct dyes, alone or as mixtures. In particular, mention may be made of direct dyes from among: azo; methine; carbonyl; azine; nitro (hetero)aryl; tri(hetero)arylmethane; porphyrin; phthalocyanine and natural direct dyes, alone or as mixtures.

When they are present, the direct dye(s) more particularly represent from 0.0001% to 10% by weight of the total weight of the dye composition and preferably from 0.005% to 5% by weight.

### Liquid fatty substances:

As has been mentioned, the composition of the invention comprises one or more liquid fatty substances (liquid at ambient temperature (25°C) and atmospheric pressure (760 mmHg or 1.013×10⁵ Pa)).

The term "*fatty substance*" is intended to mean an organic compound that is insoluble in water at ordinary temperature (25°C) and at atmospheric pressure (760 mmHg; or 1.013×10⁵ Pa) (solubility of less than 5%, preferably of less than 1% and even more preferentially of less than 0.1%). They have in their structure at least one hydrocarbon-based chain containing at least 6 carbon atoms or a sequence of at least two siloxane groups. In addition, the fatty substances are generally soluble in organic solvents under the same temperature and pressure conditions, for instance chloroform, dichloromethane, carbon tetrachloride, ethanol, benzene, toluene, tetrahydrofuran (THF), liquid petroleum jelly or decamethylcyclopentasiloxane.

The fatty substances of the invention do not contain salified carboxylic acid groups.

In particular, the fatty substances of the invention are not (poly)oxyalkylenated or (poly)glycerolated ethers.

The term "*oil*" is intended to mean a "*fatty substance*" that is liquid at ambient temperature (25°C) and at atmospheric pressure (760 mmHg or 1.013×10⁵ Pa).

The term "*non-silicone oil*" is intended to mean an oil not containing any silicon (Si) atoms and the term "*silicone oil*" is intended to mean an oil containing at least one silicon atom.

More particularly, the fatty substances are chosen from liquid C₆-C₁₆ hydrocarbons, liquid hydrocarbons containing more than 16 carbon atoms, non-silicone oils of animal origin, vegetable oils of triglyceride type, liquid synthetic triglycerides, fluoro oils, liquid fatty alcohols, liquid fatty acid and/or fatty alcohol esters other than triglycerides, silicone oils, and mixtures thereof.

It is recalled that fatty alcohols, esters and acids more particularly exhibit at least one saturated or unsaturated and linear or branched hydrocarbon-based group which comprises from 6 to 30 and better still from 8 to 30 carbon atoms and which is optionally substituted, in particular with one or more hydroxyl groups (in particular 1 to 4). If they are unsaturated, these compounds can comprise one to three conjugated or non-conjugated carbon-carbon double bonds.

As regards the liquid C₆-C₁₆ hydrocarbons, they are linear, branched or optionally cyclic, and are preferably alkanes. Mention may be made, by way of example, of hexane, cyclohexane, undecane, dodecane, isododecane, tridecane or isoparaffins, such as isohexadecane or isodecane, and mixtures thereof.

The linear or branched hydrocarbons of inorganic or synthetic origin containing more than 16 carbon atoms are preferably chosen from liquid paraffins, petroleum jelly, liquid petroleum jelly, polydecenes and hydrogenated polyisobutene such as Parleam®, and mixtures thereof.

By way of hydrocarbon-based oils of animal origin, mention may be made of perhydrosqualene.

The triglyceride oils of vegetable or synthetic origin are preferably chosen from liquid fatty acid triglycerides comprising from 6 to 30 carbon atoms, for instance heptanoic or octanoic acid triglycerides, or alternatively, for example, sunflower oil, maize oil, soya bean oil, marrow oil, grapeseed oil, sesame seed oil, hazelnut oil, apricot oil, macadamia oil, arara oil, castor oil, avocado oil, caprylic/capric acid triglycerides, for instance those sold by the company Stéarineries Dubois or those sold under the names Miglyol® 810, 812 and 818 by the company Dynamit Nobel, jojoba oil and shea butter oil, and mixtures thereof.

As regards the fluoro oils, they may be chosen from perfluoromethylcyclopentane and perfluoro-1,3-dimethylcyclohexane, sold under the names Flutec® PC1 and Flutec® PC3 by the company BNFL Fluorochemicals; perfluoro-1,2-dimethylcyclobutane; perfluoroalkanes such as dodecafluoropentane and tetradecafluorohexane, sold under the names PF 5050® and PF 5060® by the company 3M, or bromoperfluorooctyl sold under the name Foralkyl® by the company Atochem; nonafluoromethoxybutane and nonafluoroethoxyisobutane; perfluoromorpholine derivatives such as 4-trifluoromethylperfluoromorpholine sold under the name PF 5052® by the company 3M.

The liquid fatty alcohols which are suitable for the implementation of the invention are more particularly chosen from saturated or unsaturated, linear or branched alcohols comprising from 6 to 30 carbon atoms and preferably from 8 to 30 carbon atoms. Mention may be made, for example, of octyldodecanol, 2-butyloctanol, 2-hexyldecanol, 2-undecylpentadecanol, isostearyl alcohol, oleyl alcohol, linolenyl alcohol, ricinoleyl alcohol, undecylenyl alcohol or linoleyl alcohol, and mixtures thereof.

As regards the liquid fatty acid and/or fatty alcohol esters other than the triglycerides mentioned above, mention may be made in particular of esters of saturated or unsaturated, linear C₁-C₂₆ or branched C₃-C₂₆ aliphatic monoacids or polyacids and of saturated or unsaturated, linear C₁-C₂₆ or branched C₃-C₂₆ aliphatic monoalcohols or polyalcohols, the total carbon number of the esters being greater than or equal to 6 and more advantageously greater than or equal to 10.

Preferably, for the esters of monoalcohols, at least one of the alcohol or of the acid from which the esters of the invention result is branched.

Among the monoesters, mention may be made of dihydroabietyl behenate; octyldodecyl behenate; isocetyl behenate; isostearyl lactate; lauryl lactate; linoleyl lactate; oleyl lactate; isostearyl octanoate; isocetyl octanoate; octyl octanoate; decyl oleate; isocetyl isostearate; isocetyl laurate; isocetyl stearate; isodecyl octanoate; isodecyl oleate; isononyl isononanoate; isostearyl palmitate; methylacetyl ricinoleate; octyl isononanoate; 2-ethylhexyl isononanoate; octyldodecyl erucate; oleyl erucate; ethyl and isopropyl palmitates, 2-ethylhexyl palmitate, 2-octyldecyl palmitate, alkyl myristates such as isopropyl myristate, 2-octyldodecyl myristate, isobutyl stearate; 2-hexyldecyl laurate, and mixtures thereof.

Among the monoesters of monoacids and of monoalcohols, use will preferably be made of ethyl palmitate, isopropyl palmitate, alkyl myristates such as isopropyl myristate or ethyl myristate, isocetyl stearate, 2-ethylhexyl isononanoate, isodecyl neopentanoate and isostearyl neopentanoate, and mixtures thereof.

Still within the context of this variant, esters of C₄-C₂₂ dicarboxylic or tricarboxylic acids and of C₁-C₂₂ alcohols and esters of mono-, di- or tricarboxylic acids and of C₂-C₂₆ di-, tri-, tetra- or pentahydroxy alcohols may also be used.

Mention may in particular be made of: diethyl sebacate; diisopropyl sebacate; diisopropyl adipate; di(n-propyl) adipate; dioctyl adipate; diisostearyl adipate; dioctyl maleate; glyceryl undecylenate; octyldodecyl stearoyl stearate; pentaerythrityl monoricinoleate; pentaerythrityl tetraisononanoate; pentaerythrityl tetrapelargonate; pentaerythrityl tetraisostearate; pentaerythrityl tetraoctanoate; propylene glycol dicaprylate; propylene glycol dicaprate; tridecyl erucate; triisopropyl citrate; triisostearyl citrate; glyceryl trilactate; glyceryl trioctanoate; trioctyldodecyl citrate; trioleyl citrate; propylene glycol dioctanoate; neopentyl glycol diheptanoate; diethylene glycol diisononanoate; and polyethylene glycol distearates, and mixtures thereof.

The composition may also comprise, as fatty ester, sugar esters and diesters of C₆-C₃₀ and preferably C₁₂-C₂₂ fatty acids. It is recalled that the term "*sugar*" is intended to mean oxygen-comprising hydrocarbon-based compounds which have several alcohol functions, with or without an aldehyde or ketone function, and which comprise at least 4 carbon atoms. These sugars can be monosaccharides, oligosaccharides or polysaccharides.

Mention may be made, as suitable sugars, for example, of sucrose (or saccharose), glucose, galactose, ribose, fucose, maltose, fructose, mannose, arabinose, xylose and lactose, and their derivatives, in particular alkyl derivatives, such as methyl derivatives, for example methylglucose.

The esters of sugars and of fatty acids can be chosen in particular from the group consisting of the esters or mixtures of esters of sugars described above and of saturated or unsaturated, linear or branched C₆-C₃₀ and preferably C₁₂-C₂₂ fatty acids. If they are unsaturated, these compounds can comprise one to three conjugated or non-conjugated carbon-carbon double bonds.

The esters according to this alternative form can also be chosen from mono-, di-, tri- and tetraesters, polyesters and mixtures thereof.

These esters may be, for example, oleates, laurates, palmitates, myristates, behenates, cocoates, stearates, linoleates, linolenates, caprates, arachidonates or mixtures thereof, such as, in particular, oleate/palmitate, oleate/stearate or palmitate/stearate mixed esters.

More particularly, use is made of monoesters and diesters and in particular mono- or di-oleate, -stearate, -behenate, -oleopalmitate, -linoleate, -linolenate or -oleostearate of sucrose, of glucose or of methylglucose, and mixtures thereof.

An example that may be mentioned is the product sold under the name Glucate® DO by the company Amerchol, which is a methylglucose dioleate.

Use will preferably be made of a liquid ester of a monoacid and of a monoalcohol.

The silicones that may be used in the dye composition of the present invention are volatile or non-volatile, cyclic, linear or branched silicones, which are unmodified or modified by organic groups, having a viscosity from 5×10⁻⁶ to 2.5 m²/s at 25°C, and preferably 1×10⁻⁵ to 1 m²/s.

The silicones that can be used in accordance with the invention are in the form of oils.

Preferably, the silicone is chosen from liquid polydialkylsiloxanes, in particular liquid polydimethylsiloxanes (PDMSs), and liquid polyorganosiloxanes comprising at least one aryl group.

These silicones may also be organomodified. The organomodified silicones that can be used in accordance with the invention are liquid silicones as defined above and comprising in their structure one or more organofunctional groups attached by means of a hydrocarbon-based group, for example chosen from amino groups and alkoxy groups.

Organopolysiloxanes are defined in greater detail in Walter Noll's "Chemistry and Technology of Silicones" (1968), Academic Press. They may be volatile or non-volatile.

When they are volatile, the silicones are more particularly chosen from those having a boiling point of between 60°C and 260°C, and even more particularly from:
(i) cyclic polydialkylsiloxanes comprising from 3 to 7 and preferably from 4 to 5 silicon atoms. These are, for example, octamethylcyclotetrasiloxane sold in particular under the name Volatile Silicone® 7207 by Union Carbide or Silbione® 70045 V2 by Rhodia, decamethylcyclopentasiloxane sold under the name Volatile Silicone® 7158 by Union Carbide, and Silbione® 70045 V5 by Rhodia, and mixtures thereof.
   Mention may also be made of cyclocopolymers of the dimethylsiloxane/methylalkylsiloxane type, such as Volatile Silicone® FZ 3109 sold by Union Carbide, of formula: 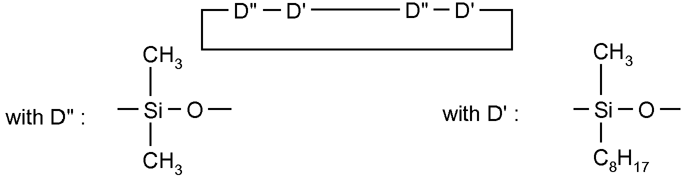
   Mention may also be made of mixtures of cyclic polydialkylsiloxanes with organic compounds derived from silicon, such as the mixture of octamethylcyclotetrasiloxane and tetra(trimethylsilyl)pentaerythritol (50/50) and the mixture of octamethylcyclotetrasiloxane and oxy-1,1'-bis(2,2,2',2',3,3'-hexatrimethylsilyloxy)neopentane;
(ii) linear volatile polydialkylsiloxanes containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, "Volatile Silicone Fluids for Cosmetics".

Non-volatile polydialkylsiloxanes are preferably used.

These silicones are more particularly chosen from polydialkylsiloxanes, among which mention may be made mainly of polydimethylsiloxanes having trimethylsilyl end groups. The viscosity of the silicones is measured at 25°C according to Standard ASTM 445 Appendix C.

Mention may be made, among these polydialkylsiloxanes, without limitation, of the following commercial products:
- the Silbione® oils of the 47 and 70 047 series or the Mirasil® oils sold by Rhodia, such as, for example, the 70 047 V 500 000 oil;
- the oils of the Mirasil® series sold by the company Rhodia;
- the oils of the 200 series from the company Dow Corning, such as DC200, having a viscosity of 60 000 mm²/s;
- the Viscasil® oils from General Electric and certain oils of the SF series (SF 96, SF 18) from General Electric.

Mention may also be made of polydimethylsiloxanes having dimethylsilanol end groups known under the name dimethiconol (CTFA), such as the oils of the 48 series from the company Rhodia.

The organomodified silicones which can be used in accordance with the invention are silicones as defined above and comprising, in their structure, one or more organofunctional groups attached by means of a hydrocarbon-based group.

As regards the liquid polyorganosiloxanes comprising at least one aryl group, they may in particular be polydiphenylsiloxanes, and polyalkylarylsiloxanes functionalized with the organofunctional groups mentioned above.

The polyalkylarylsiloxanes are particularly chosen from linear and/or branched polydimethyl/methylphenylsiloxanes and polydimethyl/diphenylsiloxanes with a viscosity ranging from 1×10⁻⁵ to 5×10⁻² m²/s at 25°C.

Mention may be made, among these polyalkylarylsiloxanes, by way of example, of the products sold under the following names:
- the Silbione® oils of the 70 641 series from Rhodia;
- the oils of the Rhodorsil® 70 633 and 763 series from Rhodia;
- the oil Dow Corning 556 Cosmetic Grade Fluid from Dow Corning;
- the silicones of the PK series from Bayer, such as the product PK20;
- the silicones of the PN and PH series from Bayer, such as the products PN1000 and PH1000;
- certain oils of the SF series from General Electric, such as SF 1023, SF 1154, SF 1250 and SF 1265.

Mention may be made, among the organomodified silicones, of polyorganosiloxanes comprising:
- substituted or unsubstituted amino groups, such as the products sold under the name GP 4 Silicone Fluid and GP 7100 by the company Genesee or the products sold under the names Q2 8220 and Dow Corning 929 or 939 by the company Dow Corning. The substituted amino groups are, in particular, C₁-C₄ aminoalkyl groups;
- alkoxy groups.

The liquid fatty substances are advantageously chosen from liquid C₆-C₁₆ alkanes, liquid hydrocarbons containing more than 16 carbon atoms, vegetable oils of triglyceride type, liquid synthetic triglycerides, liquid fatty alcohols, liquid fatty acid and/or fatty alcohol esters other than triglycerides, and mixtures thereof.

Preferably, the liquid fatty substance is chosen from liquid petroleum jelly, liquid C₆-C₁₆ alkanes, polydecenes, liquid fatty acid and/or fatty alcohol esters other than triglycerides, liquid fatty alcohols, or mixtures thereof, and even more preferentially from liquid petroleum jelly, liquid C₆-C₁₆ alkanes and polydecenes.

Of course, the composition according to the invention may comprise one or more additional fatty substances other than the liquid fatty substances which have just been described, and which are not liquid at ambient temperature and atmospheric pressure.

The composition according to the invention preferably comprises at least 10% by weight of liquid fatty substance(s), preferably at least 15% by weight, even more advantageously at least 30% by weight and up to 70% by weight relative to the total weight of the composition.

### Non-ionic guar gum

The composition according to the invention comprises at least one non-ionic guar gum. The term "non-ionic guar gum" is intended to mean modified non-ionic guar gums and unmodified non-ionic guar gums.

The unmodified non-ionic guar gums are, for example, the products sold under the name Vidogum GH 175 by the company Unipectine and under the names Meypro-Guar 50 and Jaguar C by the company Rhodia Chimie.

The modified non-ionic guar gums are in particular modified with C₁-C₆ hydroxyalkyl groups.

Among the hydroxyalkyl groups that may be mentioned, for example, are hydroxymethyl, hydroxyethyl, hydroxypropyl and hydroxybutyl groups.

These hydroxyalkylated guar gums are well known in the prior art and can be prepared, for example, by reacting corresponding alkene oxides such as, for example, propylene oxides, with the guar gum so as to obtain a guar gum modified with hydroxyalkyl groups, hydroxypropyl groups in the case of the example.

The degree of hydroxyalkylation, which corresponds to the number of alkylene oxide molecules consumed by the number of free hydroxyl functions present on the guar gum, preferably ranges from 0.4 to 1.2.

Such non-ionic guar gums optionally modified with hydroxyalkyl groups are sold, for example, under the trade names Jaguar HP8, Jaguar HP60 and Jaguar HP120, Jaguar DC 293 and Jaguar HP 105 by the company Rhodia Chimie or under the name Galactasol 4H4FD2 by the company Aqualon.

Also suitable are non-ionic guar gums modified with hydroxyalkyl groups, more especially hydroxypropyl groups, modified with groups comprising at least one C₆-C₃₀ fatty chain. By way of example of such compounds, mention may be made, *inter alia*, of the product Esaflor HM 22® (C₂₂ alkyl chain) sold by the company Lamberti, and the products RE210-18® (C₁₄ alkyl chain) and RE205-1® (C₂₀ alkyl chain) sold by the company Rhodia Chimie.

The guar gums may optionally comprise at least one C₆-C₃₀ fatty chain.

More particularly, the content of non-ionic guar gum(s) ranges from 0.001% to 10% by weight, preferably from 0.01% to 5% by weight, better still from 0.1% to 5% by weight and even more particularly from 1% to 5% by weight relative to the total weight of the composition.

### Anionic alkyl sulfate surfactants

As indicated above, the composition according to the invention comprises at least one anionic C₆-C₄₀, preferably C₆-C₂₄ and even more advantageously C₁₂-C₂₀ alkyl sulfate surfactant The alkyl group may be linear or branched.

The compounds in non-salified form can also be used in the composition according to the invention.

It should also be noted that the alkyl sulfates do not comprise an oxyethylene unit.

When the anionic surfactant(s) is (are) in salt form, it (they) may be chosen from alkali metal salts such as the sodium or potassium salt and preferably the sodium salt, ammonium salts, amine salts and in particular amino alcohol salts or alkaline-earth metal salts such as the magnesium salts.

By way of example of amino alcohol salts, mention may in particular be made of monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts, and in particular sodium or magnesium salts, are preferably used.

Among the preferred anionic alkyl sulfate surfactants, use is made of (C₆-C₂₄) alkyl sulfates, and in particular lauryl sulfate and cetyl sulfate, even better still lauryl sulfates in particular of alkali metals, of ammonium, of amino alcohols, and of alkaline-earth metals, or a mixture of these compounds.

In accordance with one advantageous embodiment of the invention, the content of anionic alkyl sulfate surfactant(s) ranges from 0.1% to 30% by weight, preferably from 0.5% to 20% by weight and more preferably from 1% to 10% by weight relative to the total weight of the composition of the invention.

### Non-ionic alkylpolyglycoside surfactants

The non-ionic alkylpolyglycoside surfactants present in the composition according to the invention are surfactants well-known from the prior art. These surfactants may be represented more particularly by the following general formula:

R₁O-(R₂O)ₜ(G)ᵥ

in which R₁ represents a linear or branched alkyl and/or alkenyl radical comprising approximately from 8 to 24 carbon atoms, an alkylphenyl radical in which the linear or branched alkyl radical comprises from 8 to 24 carbon atoms, R₂ represents an alkylene radical comprising approximately from 2 to 4 carbon atoms, G represents a sugar unit comprising from 5 to 6 carbon atoms, t denotes a value ranging from 0 to 10, preferably from 0 to 4, preferably from 0 to 3, and v denotes a value ranging from 1 to 15, preferably from 1 to 4.

According to one particular embodiment, the alkylpolyglycoside surfactants are compounds having the formula described above in which R₁ more particularly denotes a linear or branched, saturated or unsaturated alkyl radical comprising from 8 to 18 carbon atoms, t denotes a value ranging from 0 to 3 and even more particularly equal to 0, and G may denote glucose, fructose or galactose, preferably glucose. The degree of polymerization, i.e. the value of v in the formula above, may range from 1 to 15 and preferably from 1 to 4. The average degree of polymerization is more particularly between 1 and 2.

The glucosidic bonds between the sugar units are of 1-6 or 1-4 type and preferably of 1-4 type.

Compounds corresponding to the formula above are in particular represented by the products sold by the company Cognis under the name Plantaren® (600 CS/U, 1200 and 2000) or Plantacare® (818, 1200 and 2000). It is also possible to use the products sold by the company SEPPIC under the names Triton CG110 (or Oramix CG 10) and Triton CG312 (or Oramix® NS 10), the products sold by the company BASF under the name Lutensol GD 70 or those sold by the company Chem Y under the name AG10 LK.

Mention may also be made of the C8/C16 alkyl 1,4-polyglucoside as an aqueous 53% solution sold by Cognis under the reference Plantacare® 818 UP.

According to one particular embodiment of the invention, the content of non-ionic alkylpolyglycoside surfactant(s) ranges from 0.1% to 30% by weight, preferably from 1% to 20% by weight and more preferably from 1% to 10% by weight, relative to the total weight of the composition.

Preferably, the non-ionic alkylpolyglycoside surfactant(s)/anionic alkyl sulfate surfactant(s) weight ratio ranges from 0.1 to 10 and better still from 1 to 10.

Even more preferentially, this ratio is greater than or equal to 1.5 and even better still ranges from 1.5 to 5.

### Alkaline agent

The composition according to the invention advantageously comprises at least one alkaline agent.

This agent may be chosen from inorganic or organic or hybrid alkaline agents, or mixtures thereof.

The inorganic alkaline agent(s) are preferably chosen from aqueous ammonia, alkali carbonates or bicarbonates such as sodium or potassium carbonates and sodium or potassium bicarbonates, sodium hydroxide or potassium hydroxide, or mixtures thereof.

The organic alkaline agent(s) are preferably chosen from organic amines with a pKb at 25°C of less than 12, preferably less than 10 and even more advantageously less than 6. It should be noted that it is the pKb corresponding to the function of highest basicity.

Hybrid compounds that may be mentioned include the salts of the amines mentioned previously with acids such as carbonic acid or hydrochloric acid.

The organic alkaline agent(s) are chosen, for example, from alkanolamines, oxyethylenated and/or oxypropylenated ethylenediamines, amino acids and the compounds having the formula below: 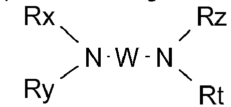 in which W is a C₁-C₆ alkylene residue optionally substituted with a hydroxyl group or a C₁-C₆ alkyl radical; Rx, Ry, Rz and Rt, which may be identical or different, represent a hydrogen atom or a C₁-C₆ alkyl, C₁-C₆ hydroxyalkyl or C₁-C₆ aminoalkyl radical.

Examples of such amines that may be mentioned include 1,3-diaminopropane, 1,3-diamino-2-propanol, spermine and spermidine.

The term "alkanolamine" is intended to mean an organic amine comprising a primary, secondary or tertiary amine function, and one or more linear or branched C₁-C₈ alkyl groups bearing one or more hydroxyl radicals.

Alkanolamines such as monoalkanolamines, dialkanolamines or trialkanolamines comprising from one to three identical or different C₁-C₄ hydroxyalkyl radicals are in particular suitable for implementing the invention.

Among compounds of this type, mention may be made of monoethanolamine, diethanolamine, triethanolamine, monoisopropanolamine, diisopropanolamine, N-dimethylaminoethanolamine, 2-amino-2-methyl-1-propanol, triisopropanolamine, 2-amino-2-methyl-1,3-propanediol, 3-amino-1,2-propanediol, 3-dimethylamino-1,2-propanediol and tris(hydroxymethylamino)methane.

More particularly, the amino acids that may be used are of natural or synthetic origin, in their L, D or racemic form, and comprise at least one acid function chosen more particularly from carboxylic acid, sulfonic acid, phosphonic acid or phosphoric acid functions. The amino acids may be in neutral or ionic form.

Mention may in particular be made, as amino acids which can be used in the present invention, of aspartic acid, glutamic acid, alanine, arginine, ornithine, citrulline, asparagine, carnitine, cysteine, glutamine, glycine, histidine, lysine, isoleucine, leucine, methionine, N-phenylalanine, proline, serine, taurine, threonine, tryptophan, tyrosine and valine.

Advantageously, the amino acids are basic amino acids comprising an additional amine function optionally included in a ring or in a ureido function.

Such basic amino acids are chosen, for example, from histidine, lysine, arginine, ornithine, and citrulline.

The organic amine may also be chosen from organic amines of heterocyclic type. Besides histidine that has already been mentioned in the amino acids, mention may be made in particular of pyridine, piperidine, imidazole, triazole, tetrazole and benzimidazole.

The organic amine can also be chosen from amino acid dipeptides. As amino acid dipeptides that may be used in the present invention, mention may be made in particular of carnosine, anserine and balenine.

The organic amine is chosen from compounds comprising a guanidine function. As amines of this type that may be used in the present invention, besides arginine, which has already been mentioned as an amino acid, mention may be made in particular of creatine, creatinine, 1,1-dimethylguanidine, 1,1-diethylguanidine, glycocyamine, metformin, agmatine, N-amidinoalanine, 3-guanidinopropionic acid, 4-guanidinobutyric acid and 2-([amino(imino)methyl]amino)ethane-1-sulfonic acid.

As hybrid compounds, mention may be made in particular of guanidine carbonate or monoethanolamine hydrochloride.

More particularly, the dye composition used in the process of the invention contains, as alkaline agent, aqueous ammonia and/or at least one alkanolamine and/or at least one basic amino acid, more advantageously aqueous ammonia and/or at least one alkanolamine. Preferably, the alkaline agent is chosen from aqueous ammonia and monoethanolamine, or mixtures thereof. Even more preferentially, the alkaline agent is an alkanolamine and better still is monoethanolamine.

Advantageously, the composition has a content of alkaline agent(s), and preferably of organic amine, ranging from 0.01% to 30% by weight, preferably from 0.1% to 20% by weight and better still from 1% to 10% by weight, relative to the weight of said dye composition. It should be noted that this content is expressed as NH₃ when the alkaline agent is aqueous ammonia.

Preferably, the composition of the invention does not contain enzymes.

### Additives

The composition may also contain various adjuvants conventionally used in compositions for dyeing or lightening the hair, such as anionic, cationic or non-ionic polymers other than non-ionic guar gums, or mixtures thereof; surfactants other than those used in the composition of the invention and in particular cationic or amphoteric surfactants or non-ionic surfactants other than alkylpolyglycosides or anionic surfactants other than alkyl sulfates; antioxidants; penetrating agents; sequestrants; fragrances; dispersants; film-forming agents; ceramides; preservatives; opacifiers.

If they are present, the above additives are generally present in an amount for each of them of between 0.01% and 20% by weight, relative to the weight of the composition.

The composition according to the invention may comprise water and/or one or more organic solvents.

Examples of organic solvents that may be mentioned include linear or branched and preferably saturated monoalcohols or diols, comprising 2 to 6 carbon atoms, such as ethyl alcohol, isopropyl alcohol, hexylene glycol (2-methyl-2,4-pentanediol), neopentyl glycol and 3-methyl-1,5-pentanediol, butylene glycol, dipropylene glycol and propylene glycol; aromatic alcohols such as benzyl alcohol or phenylethyl alcohol; polyols containing more than two hydroxyl functions, such as glycerol; polyol ethers, for instance ethylene glycol monomethyl, monoethyl and monobutyl ethers, propylene glycol or ethers thereof, for instance propylene glycol monomethyl ether; and also diethylene glycol alkyl ethers, in particular C₁-C₄ alkyl ethers, for instance diethylene glycol monoethyl ether or monobutyl ether, alone or as a mixture.

The organic solvents, when they are present, generally represent between 1% and 40% by weight relative to the total weight of the dye composition, and preferably between 5% and 30% by weight relative to the total weight of the dye composition.

The composition is preferably aqueous. In this case, it preferably comprises from 30% to 95% by weight of water, better still from 40% to 90% by weight of water and even better still from 50% to 85% by weight of water relative to the total weight of the composition.

The pH of the composition according to the invention, if it is aqueous, generally ranges from 6 to 12 and preferentially from 8.5 to 11.5.

It may be adjusted to the desired value by means of acidifying or basifying agents normally used in the dyeing of keratin fibres.

### Dyeing process

The dyeing process according to the invention consists in applying the composition of the invention to human keratin fibres in the presence of at least one chemical oxidizing agent other than atmospheric oxygen.

In particular, the formulation applied results from the extemporaneous mixing, before use, of the composition according to the invention with an oxidizing composition comprising at least one chemical oxidizing agent other than atmospheric oxygen.

### Oxidizing composition

The oxidizing composition comprises at least one chemical oxidizing agent other than atmospheric oxygen.

More particularly, the chemical oxidizing agent(s) is (are) chosen from hydrogen peroxide, urea peroxide, alkali metal bromates or ferricyanides, peroxygenated salts, for instance persulfates, perborates, peracids and precursors thereof and alkali metal or alkaline-earth metal percarbonates. Advantageously, the oxidizing agent is hydrogen peroxide.

Preferably, the oxidizing agent is not chosen from enzymes.

Preferably, the oxidizing composition comprises hydrogen peroxide as chemical oxidizing agent other than atmospheric oxygen, in aqueous solution, the concentration of which ranges, more particularly, from 0.1% to 50%, more particularly between 0.5% and 20% and even more preferentially between 1% and 15% by weight, relative to the weight of the oxidizing composition.

Usually, the pH of the oxidizing composition is less than 7.

It can be adjusted by adding acidifying agents, such as hydrochloric acid, (ortho)phosphoric acid, sulfuric acid, boric acid, and also carboxylic acids, for instance acetic acid, lactic acid or citric acid, or sulfonic acids. Alkaline agents such as those previously mentioned may also be used.

The composition may also comprise water and/or one or more organic solvents such as those listed previously in the context of the description of the composition according to the invention and to which reference may be made.

The organic solvents, when they are present, generally represent between 1% and 40% by weight relative to the total weight of the composition, and preferably between 5% and 30% by weight relative to the total weight of the composition.

In accordance with one particular variant, the oxidizing composition may comprise at least one fatty substance, preferably liquid fatty substance. Reference may be made to the description that was previously given for the description of the composition according to the invention.

Preferably, if the oxidizing composition comprises at least one fatty substance, preferably liquid fatty substance, then the content of fatty substance is at least 5% by weight, even more preferentially at least 10% by weight and better still at least 15% by weight of fatty substance relative to the weight of the oxidizing composition.

The composition may also contain various adjuvants conventionally used in compositions for dyeing or lightening the hair, such as cationic, anionic or non-ionic polymers, or mixtures thereof; surfactants; antioxidants; penetrating agents; sequestrants; fragrances; dispersants; film-forming agents; ceramides; preservatives; opacifiers.

If they are present, the above additives are generally present in an amount for each of them of between 0.01% and 20% by weight relative to the weight of the composition.

In one variant of the invention, the composition of the invention is applied to the fibres in the presence of at least one chemical oxidizing agent other than atmospheric oxygen, in the form of a foam.

The composition in foam form is formed from a mixture of air or an inert gas with the composition described previously.

According to this embodiment, the composition may be packaged in a foam dispenser.

It may be a case of "aerosol" products dispensed from a pressurized container by means of a propellant gas and thus forming a foam at the moment when they are dispensed.

The propellant gas(es), which may or may not be identical, that may be used may be chosen from carbon dioxide, nitrogen, nitrogen oxide, dimethyl ether, volatile hydrocarbons such as butane, isobutane, propane or pentane, and mixtures thereof.

It may also be a case of products dispensed from a container by means of a mechanical pump connected to a dispensing head, the passage of the composition through the dispensing head transforming the composition into foam at the very latest at the outlet orifice of such a head.

Finally, the foam can be generated by shaking the composition according to the invention in a container closed with a lid, such as a shaker.

In another variant of the invention, the composition according to the invention and the mixture resulting from the composition of the invention and from a composition comprising at least one chemical oxidizing agent other than atmospheric oxygen constitute a cream or a cream gel.

The dyeing process according to the invention therefore consists in applying to dry or wet human keratin fibres the mixture obtained, just before application, from the composition according to the invention and from a composition comprising at least one chemical oxidizing agent other than atmospheric oxygen.

The composition resulting from the mixture is left in place for a time sufficient to develop the desired colouration.

The dyeing process is generally performed at ambient temperature (between 15 and 25°C) and up to temperatures that may be as high as 60°C to 80°C.

After a leave-on time of from one minute to one hour and preferably from 5 minutes to 30 minutes, the keratin fibres are rinsed with water, optionally washed with a shampoo and then rinsed with water.

The examples that follow serve to illustrate the invention without, however, being limiting in nature.

### EXAMPLE 1:

The following compositions are prepared (the amounts are expressed in g% of active material):

**Composition 1:**

| **Ingredients** | **Content** |
|---|---|
| Resorcinol | 0.66 |
| Ethanolamine | 5.35 |
| Caprylyl/capryl glucoside | 3 |
| Sodium lauryl sulfate | 1.24 |
| Hydroxypropyl guar | 1 |
| Ascorbic acid | 0.12 |
| m-Aminophenol | 0.12 |
| EDTA | 0.2 |
| 2-Methylresorcinol | 0.038 |
| PEG-40 hydrogenated castor oil | 1 |
| Sodium chloride | 0.65 |
| Sodium metabisulfite | 0.22 |
| Mineral oil | 60 |
| 2,5-Toluenediamine | 0.718 |
| Water | qs 100 |

**Composition 2:**

| **Ingredients** | **Content** |
|---|---|
| Tocopherol | 0.1 |
| Sodium stannate | 0.04 |
| Pentasodium pentetate | 0.06 |
| Polyquaternium-6 | 0.2 |
| Cetearyl alcohol | 6 |
| Hexamethrine chloride | 0.15 |
| Glycerol | 0.5 |
| Hydrogen peroxide | 6 |
| Tetrasodium pyrophosphate | 0.03 |
| Mineral oil | 20 |
| PEG-4 rapeseed amide | 1.19 |
| Steareth-20 | 5 |
| Phosphoric acid | qs pH 2.2 |
| Water | qs |

### Application method:

The two compositions are mixed together at the time of use in the following proportions: 10 g of composition 1 with 15 g of composition 2.

The resulting composition is a cream which has a foaming nature. It is easily applied to locks of grey hair containing 90% white hairs, in a proportion of 10 g of mixture per 1 g of hair.

It is left for 30 minutes at ambient temperature (20°C).

The hair is then rinsed, washed with a standard shampoo and dried.

A powerful and homogeneous dark blonde colouration (good uptake of the colouration) is obtained.

### EXEMPLE 2:

The following compositions are prepared (the amounts are expressed in g% of active material):

| | A (invention) | B (comparative) |
|---|---|---|
| Resorcinol | 0,66 | 0,66 |
| m-Aminophenol | 0,12 | 0,12 |
| 2-Methylresorcinol | 0,038 | 0,038 |
| 2,5-Toluenediamine | 0,718 | 0,718 |
| Monoethanolamine | 5,35 | 5,35 |
| Caprylyl/capryl glucoside | 3 | 3 |
| Sodium Lauryl sulfate | 1,24 | 1,24 |
| Hydroxypropyl guar | 1 | 1 |
| PEG-40 hydrogenated castor oil | 1 | 1 |
| Mineral oil | 20 | - |
| Ascorbic acid | 0,12 | 0,12 |
| Sodium metabisulfite | 0,22 | 0,22 |
| EDTA | 0,2 | 0,2 |
| Sodium chloride | 0,65 | 0,65 |
| Water | Qs 100 | Qs 100 |

### Application method:

At the time of use, each of the above compositions was mixed with a 20-volumes hydrogen peroxide solution (1 part by weight of the compositions with 1 part and a half by weight of a 20-volumes hydrogen peroxide solution, 6g% of H2O2).

Each of the resulting mixtures was then applied onto locks of permed hair containing 90% of white hair. 10g of the mixture is applied for 1g of hair.

After 30 minutes at ambient temperature, the hair was then rinsed, washed with a standard shampoo and dried.

### Result:

The color of the hair was determined by using a DATACOLOR SF600X spectrophotometer (D65, 10°, specular components included) in the L*a*b* system.

According to this system, L* indicates the lightness of the color of the hair. The lowest is the value of L, the most intense is the color of the hair.

| | L* |
|---|---|
| A (invention) | 23,7 |
| B (comparative) | 27,6 |

These results show that the hair treated with composition A has a value of L* lower than the hair treated with composition B. So, composition of the invention A provides higher coloration power, in term of intensity, than comparative composition B.

## Claims

1. Composition for dyeing human keratin fibres which is free of chemical oxidizing agent other than atmospheric oxygen and which comprises:
(a) at least one oxidation dye precursor;
(b) at least one liquid fatty substance;
(c) at least one non-ionic guar gum;
(d) at least one anionic surfactant chosen from C₆-C₄₀ alkyl sulfates or non-salified forms thereof;
(e) at least one non-ionic alkylpolyglycoside surfactant.

2. Composition according to Claim 1, in which the liquid fatty substance(s) are chosen from liquid C₆-C₁₆ hydrocarbons, liquid hydrocarbons containing more than 16 carbon atoms, non-silicone oils of animal origin, vegetable oils of triglyceride type, liquid synthetic triglycerides, fluoro oils, liquid fatty alcohols, liquid fatty acid and/or fatty alcohol esters other than triglycerides, and silicone oils.

3. Composition according to Claim 1 or 2, in which the liquid fatty substance(s) are chosen from liquid petroleum jelly, liquid C₆-C₁₆ alkanes, polydecenes, liquid fatty acid and/or fatty alcohol esters other than triglycerides, liquid fatty alcohols, or mixtures thereof, and even more preferentially from liquid petroleum jelly, liquid C₆-C₁₆ alkanes and polydecenes, or mixtures thereof.

4. Composition according to any one of the preceding claims, in which the concentration of liquid fatty substances is at least 10% by weight, preferably at least 15% by weight, even more advantageously at least 30% by weight and up to 70% by weight relative to the total weight of the composition.

5. Composition according to any one of the preceding claims, in which the non-ionic guar gum is unmodified or modified with C₁-C₆ hydroxyalkyl groups, optionally comprising groups comprising at least one C₆-C₃₀ fatty chain.

6. Composition according to any one of the preceding claims, in which the content of non-ionic guar gum(s) ranges from 0.001 % to 10% by weight, preferably from 0.01% to 5% by weight, better still from 0.1% to 5% by weight and even better still from 1% to 5% by weight relative to the total weight of the composition.

7. Composition according to any one of the preceding claims, in which the dye precursor is chosen from oxidation bases and couplers.

8. Composition according to any one of the preceding claims, in which the composition contains at least one oxidation base.

9. Composition according to any one of the preceding claims, in which the anionic alkyl sulfate surfactant(s) are chosen from C₆-C₂₄ and in particular C₁₂-C₂₀ alkyl sulfates, in particular of alkali metals, of ammonium, of amino alcohols, and of alkaline-earth metals, or a mixture of these compounds.

10. Composition according to any one of the preceding claims, in which the content of anionic alkyl sulfate surfactant(s) ranges from 0.1% to 30% by weight, preferably from 0.5% to 20% by weight and more preferably from 1% to 10% by weight, relative to the total weight of the composition.

11. Composition according to any one of the preceding claims, in which the non-ionic alkylpolyglycoside surfactant is chosen from those having the following general formula:
R₁O-(R₂O)ₜ(G)ᵥ
in which R₁ represents a linear or branched alkyl and/or alkenyl radical comprising approximately from 8 to 24 carbon atoms, an alkylphenyl radical in which the linear or branched alkyl radical comprises from 8 to 24 carbon atoms, R₂ represents an alkylene radical comprising approximately from 2 to 4 carbon atoms, G represents a sugar unit comprising from 5 to 6 carbon atoms, t denotes a value ranging from 0 to 10, preferably from 0 to 4, preferably from 0 to 3, and v denotes a value ranging from 1 to 15.

12. Composition according to Claim 11, in which the alkylpolyglycoside surfactant(s) are such that R₁ denotes a linear or branched, saturated or unsaturated alkyl radical comprising from 8 to 18 carbon atoms, t denotes a value ranging from 0 to 3 and even more particularly equal to 0, and G may denote glucose, fructose or galactose, preferably glucose, v ranging from 1 to 15, preferably from 1 to 4, more particularly from 1 to 2.

13. Composition according to any one of the preceding claims, in which the content of non-ionic alkylpolyglycoside surfactant(s) ranges from 0.1% to 30% by weight, preferably from 1% to 20% by weight and more preferably from 1% to 10% by weight, relative to the total weight of the composition.

14. Composition according to any one of the preceding claims, in which the non-ionic alkylpolyglycoside surfactant(s)/anionic alkyl sulfate surfactant(s) weight ratio is greater than or equal to 1.5 and preferably ranges between 1.5 and 5.

15. Process for dyeing human keratin fibres, in which a composition according to any one of Claims 1 to 14 is applied to the fibres in the presence of at least one chemical oxidizing agent other than atmospheric oxygen.

16. Dyeing process according to Claim 15, in which the chemical oxidizing agent is hydrogen peroxide.

## Patentansprüche

1. Zusammentsetzung zum Färben von menschlichen Keratinfasern, die frei von chemischen Oxidationsmitteln, die von Luftsauerstoff verschieden sind, ist und Folgendes umfasst:
(a) mindestens eine Oxidationsfarbstoff-Vorstufe;
(b) mindestens eine flüssige Fettsubstanz;
(c) mindestens ein nichtionisches Guar-Gummi;
(d) mindestens ein anionisches Tensid, das aus C₆-C₄₀-Alkylsulfaten oder unversalzten Formen davon ausgewählt ist;
(e) mindestens ein nichtionisches Alkylpolyglycosid-Tensid.

2. Zusammensetzung nach Anspruch 1, wobei die flüssige Fettsubstanz bzw. die flüssigen Fettsubstanzen aus flüssigen C₆-C₁₆-Kohlenwasserstoffen, flüssigen Kohlenwasserstoffen mit mehr als 16 Kohlenstoffatomen, Nichtsilikonölen tierischer Herkunft, Pflanzenölen vom Triglycerid-Typ, flüssigen synthetischen Triglyceriden, Fluorölen, flüssigen Fettalkoholen, flüssigen Fettsäure- und/oder Fettalkoholestern, die von Triglyceriden verschieden sind, und Silikonölen ausgewählt sind.

3. Zusammensetzung nach Anspruch 1 oder 2, bei dem die flüssige Fettsubstanz bzw. die flüssigen Fettsubstanzen aus Vaselineöl, flüssigen C₆-C₁₆-Alkanen, Polydecenen, flüssigen Fettsäure- und/oder Fettalkoholestern, die von Triglyceriden verschieden sind, flüssigen Fettalkoholen oder Mischungen davon und noch weiter bevorzugt aus Vaselineöl, flüssigen C₆-C₁₆-Alkanen und Polydecenen oder Mischungen davon ausgewählt ist bzw. sind.

4. Zusammensetzung nach einem der vorhergehenden Ansprüche, wobei die Konzentration von flüssigen Fettsubstanzen mindestens 10 Gew.-%, vorzugsweise mindestens 15 Gew.-%, noch vorteilhafter mindestens 30 Gew.-% und bis zu 70 Gew.-%, bezogen auf das Gesamtgewicht der Zusammensetzung, beträgt.

5. Zusammensetzung nach einem der vorhergehenden Ansprüche, wobei das nichtionische Guar-Gummi unmodifiziert oder mit C₁-C₆-Hydroxyalkylgruppen, die gegebenenfalls Gruppen mit mindestens einer C₆-C₃₀-Fettkette umfassen, modifiziert ist.

6. Zusammensetzung nach einem der vorhergehenden Ansprüche, wobei der Gehalt an nichtionischem Guar-Gummi bzw. nichtionischen Guar-Gummen im Bereich von 0,001 bis 10 Gew.-%, vorzugsweise von 0,01 bis 5 Gew.-%, noch besser von 0,1 bis 5 Gew.-% und noch besser von 1 bis 5 Gew.-%, bezogen auf das Gesamtgewicht der Zusammensetzung, liegt.

7. Zusammensetzung nach einem der vorhergehenden Ansprüche, wobei die Farbstoff-Vorstufe aus Oxidationsbasen und Kupplern ausgewählt ist.

8. Zusammensetzung nach einem der vorhergehenden Ansprüche, wobei die Zusammensetzung mindestens eine Oxidationsbase enthält.

9. Zusammensetzung nach einem der vorhergehenden Ansprüche, wobei das anionische Alkylsulfat-Tensid bzw. die anionischen Alkylsulfat-Tenside aus C₆-C₂₄- und insbesondere C₁₂-C₂₀-Alkylsulfaten, insbesondere von Alkalimetallen, von Ammonium, von Aminoalkoholen und von Erdalkalimetallen, oder einer Mischung davon ausgewählt ist bzw. sind.

10. Zusammensetzung nach einem der vorhergehenden Ansprüche, wobei der Gehalt an anionischem Alkylsulfat-Tensid bzw. anionischen Alkylsulfat-Tensiden im Bereich von 0,1 bis 30 Gew.-%, vorzugsweise von 0,5 bis 20 Gew.-% und weiter bevorzugt von 1 bis 10 Gew.-%, bezogen auf das Gesamtgewicht der Zusammensetzung, liegt.

11. Zusammensetzung nach einem der vorhergehenden Ansprüche, wobei das nichtionische Alkylpolyglycosid-Tensid aus denjenigen mit der folgenden allgemeinen Formel ausgewählt ist:
R₁O-(R₂O)ₜ(G)ᵥ
wobei R₁ für einen linearen oder verzweigten Alkyl- und/oder Alkenylrest mit ungefähr 8 bis 24 Kohlenstoffatomen oder einen Alkylphenylrest, in dem der lineare oder verzweigte Alkylrest 8 bis 24 Kohlenstoffatome enthält, steht, R₂ für einen Alkylrest mit 2 bis 4 Kohlenstoffatomen steht, G für eine Zuckereinheit mit 5 bis 6 Kohlenstoffatomen steht, t für einen Wert im Bereich von 0 bis 10, vorzugsweise von 0 bis 4, vorzugsweise von 0 bis 3, steht und v für einen Wert im Bereich von 1 bis 15 steht.

12. Zusammensetzung nach Anspruch 11, wobei das nichtionische Alkylpolyglycosid-Tensid bzw. die nichtionischen Alkylpolyglycosid-Tenside so beschaffen sind, dass R₁ für einen linearen oder verzweigten, gesättigten oder ungesättigten Alkylrest mit 8 bis 18 Kohlenstoffatomen steht, t für einen Wert im Bereich von 0 bis 3 und noch spezieller gleich 0 steht und G für Glucose, Fructose oder Galactose, vorzugsweise Glucose, stehen kann, wobei v im Bereich von 1 bis 15, vorzugsweise von 1 bis 4, spezieller von 1 bis 2, liegt.

13. Zusammensetzung nach einem der vorhergehenden Ansprüche, wobei der Gehalt an nichtionischem Alkylpolyglycosid-Tensid bzw. nichtionischen Alkylpolyglycosid-Tensiden im Bereich von 0,1 bis 30 Gew.-%, vorzugsweise von 1 bis 20 Gew.-% und weiter bevorzugt von 1 bis 10 Gew.-%, bezogen auf das Gesamtgewicht der Zusammensetzung, liegt.

14. Zusammensetzung nach einem der vorhergehenden Ansprüche, wobei das Gewichtsverhältnis von nichtionischem Alkylpolyglycosid-Tensid bzw. nichtionischen Alkylpolyglycosid-Tensiden zu anionischem Alkylsulfat-Tensid bzw. anionischen Alkylsulfat-Tensiden größer oder gleich 1,5 ist und vorzugsweise zwischen 1,5 und 5 liegt.

15. Verfahren zum Färben von menschlichen Keratinfasern, bei dem man eine Zusammensetzung nach einem der Ansprüche 1 bis 14 in Gegenwart von mindestens einem chemischen Oxidationsmittel, das von Luftsauerstoff verschieden ist, auf die Fasern aufbringt.

16. Färbeverfahren nach Anspruch 15, wobei es sich bei dem chemischen Oxidationsmittel um Wasserstoffperoxid handelt.

## Revendications

1. Composition destinée à la coloration des fibres kératiniques humaines, qui est exempte d'agent oxydant chimique autre que l'oxygène atmosphérique, et comprenant :
(a) au moins un précurseur de colorant d'oxydation ;
(b) au moins une substance grasse liquide ;
(c) au moins une gomme guar non ionique ;
(d) au moins un agent tensioactif anionique choisi parmi les C₆-C₄₀-alkylsulfates ou leurs formes non salifiées ;
(e) au moins un agent tensioactif non ionique à base d'alkylpolyglycoside.

2. Composition selon la revendication 1, dans laquelle la ou les substances grasses liquides sont choisies parmi les hydrocarbures liquides en C₆-C₁₆, les hydrocarbures liquides contenant plus de 16 atomes de carbone, les huiles non siliconées d'origine animale, les huiles végétales de type triglycérides, les triglycérides synthétiques liquides, les huiles fluorées, les alcools gras liquides, les esters liquides d'acides gras et/ou d'alcools gras autres que les triglycérides, et les huiles de silicone.

3. Composition selon la revendication 1 ou 2, dans laquelle la ou les substances grasses liquides sont choisies parmi l'huile de vaseline, les alcanes liquides en C₆-C₁₆, les polydécènes, les esters liquides d'acides gras et/ou d'alcools gras autres que les triglycérides, les alcools gras liquides ou des mélanges de ceux-ci, et encore plus préférablement parmi l'huile de vaseline, les alcanes liquides en C₆-C₁₆ et les polydécènes, ou des mélanges de ceux-ci.

4. Composition selon l'une quelconque des revendications précédentes, dans laquelle la concentration en substances grasses liquides est d'au moins 10% en poids, de préférence d'au moins 15% en poids, de manière encore plus avantageuse d'au moins 30% en poids et jusqu'à 70% en poids par rapport au poids total de la composition.

5. Composition selon l'une quelconque des revendications précédentes, dans laquelle la gomme guar non ionique est non modifiée ou modifiée par des groupements C₁-C₆-hydroxyalkyle, comprenant éventuellement des groupements comportant au moins une chaîne grasse en C₆-C₃₀.

6. Composition selon l'une quelconque des revendications précédentes, dans laquelle la teneur en gomme(s) guar non ionique(s) va de 0,001% à 10% en poids, de préférence de 0,01% à 5% en poids, mieux encore de 0,1% à 5% en poids, et toujours mieux encore de 1% à 5% en poids par rapport au poids total de la composition.

7. Composition selon l'une quelconque des revendications précédentes, dans laquelle le précurseur de colorant est choisi parmi les bases d'oxydation et les coupleurs.

8. Composition selon l'une quelconque des revendications précédentes, où la composition contient au moins une base d'oxydation.

9. Composition selon l'une quelconque des revendications précédentes, dans laquelle le ou les agents tensioactifs anioniques à base d'alkylsulfate sont choisis parmi les alkylsulfates en C₆-C₂₄, et en particulier en C₁₂-C₂₀, notamment de métaux alcalins, d'ammonium, d'aminoalcools, et de métaux alcalino-terreux, ou un mélange de ces composés.

10. Composition selon l'une quelconque des revendications précédentes, dans laquelle la teneur en agent(s) tensioactif(s) anionique(s) à base d'alkyl-sulfate va de 0,1% à 30% en poids, de préférence de 0,5% à 20% en poids, et plus préférablement de 1% à 10% en poids, par rapport au poids total de la composition.

11. Composition selon l'une quelconque des revendications précédentes, dans laquelle l'agent tensioactif non ionique à base d'alkylpolyglycoside est choisi parmi ceux répondant à la formule générale suivante :
R₁O-(R₂O)ₜ(G)ᵥ
où R₁ représente un radical alkyle et/ou alcényle linéaire ou ramifié comprenant environ de 8 à 24 atomes de carbone, un radical alkylphényle dont le radical alkyle linéaire ou ramifié comprend de 8 à 24 atomes de carbone, R₂ représente un radical alkylène comprenant environ de 2 à 4 atomes de carbone, G représente un motif de sucre comprenant de 5 à 6 atomes de carbone, t désigne une valeur allant de 0 à 10, de préférence de 0 à 4, de préférence de 0 à 3, et v désigne une valeur allant de 1 à 15.

12. Composition selon la revendication 11, dans laquelle le ou les agents tensioactifs à base d'alkylpolyglucoside sont tels que R₁ désigne un radical alkyle saturé ou insaturé, linéaire ou ramifié comprenant de 8 à 18 atomes de carbone, t désigne une valeur allant de 0 à 3 et encore plus particulièrement égale à 0, et G peut désigner le glucose, le fructose ou le galactose, de préférence le glucose, v variant de 1 à 15, de préférence de 1 à 4, plus particulièrement de 1 à 2.

13. Composition selon l'une quelconque des revendications précédentes, dans laquelle la teneur en agent(s) tensioactif(s) non ionique(s) à base d'alkylpolyglycoside varie de 0,1% à 30% en poids, de préférence de 1% à 20% en poids, et plus préférablement de 1% à 10% en poids, par rapport au poids total de la composition.

14. Composition selon l'une quelconque des revendications précédentes, dans laquelle le rapport pondéral agent(s) tensioactif(s) non ionique(s) à base d'alkylpolyglucoside/agent(s) tensioactif(s) anionique(s) à base d'alkylsulfate est supérieur ou égal à 1,5, et est compris de préférence entre 1,5 et 5.

15. Procédé de coloration des fibres kératiniques humaines, dans lequel on applique sur les fibres une composition selon l'une quelconque des revendications 1 à 14 en présence d'au moins un agent oxydant chimique autre que l'oxygène atmosphérique.

16. Procédé de coloration selon la revendication 15, dans lequel l'agent oxydant chimique est le peroxyde d'hydrogène.
